# EUROPÄISCHE PATENTANMELDUNG

(11) **EP 1 454 587 A2**
(43) Veröffentlichungstag der Anmeldung: **08.09.2004**
(21) Anmeldenummer: 04001059.7
(22) Anmeldetag: 20.01.2004
(51) Int. Cl.: A61B 17/00, A61B 17/11

(54) **Verschlussvorrichtung für einen Stichkanal und Applikatorvorrichtung**

(30) Priorität: 06.03.2003 DE 10310995
(71) Anmelder: Aesculap AG & Co. KG, 78532 Tuttlingen (DE)
(72) Erfinder: Müller, Gottfried, Prof. Dr., 97999 Igersheim (DE); Beck, Thomas, 78591 Durchhausen (DE); Dworschak, Manfred, 78589 Dürbheim (DE); Eggerstedt, Sven, Dr., 78532 Tuttlingen (DE)
(74) Vertreter: HOEGER, STELLRECHT & PARTNER Patentanwälte

(57) **Zusammenfassung**

Um eine Verschlußvorrichtung für eine Öffnung in einer Gewebeschicht, mit einer Mehrzahl von Flügeln, welche Anlageflächen an die Öffnung umgebendes Gewebe bereitstellen und/oder halten, so zu verbessern, wird vorgeschlagen, eine Verschlußvorrichtung der eingangs genannten Art zu schaffen, welche für einen Operateur auf einfache Weise einsetzbar ist, daß die Flügel über jeweilige Gelenke schwenkbar an einem Basisteil gehalten sind.

## Beschreibung

Die Erfindung betrifft eine Verschlußvorrichtung für eine Öffnung in einer Gewebeschicht, mit einer Mehrzahl von Flügeln, welche Anlageflächen an die Öffnung umgebendes Gewebe bereitstellen und/oder halten.

Ferner betrifft die Erfindung eine Applikatorvorrichtung für eine solche Verschlußvorrichtung.

Aus der DE 100 27 186 A1 ist eine Vorrichtung zum Verschließen eines Stichkanals in einer Gewebeschicht mit einem chirurgischen Nähfaden bekannt, welche ein flächiges zusammenfaltbares Anlageelement umfaßt, an dem der Nähfaden derart angreift, daß sich das Anlageelement bei Zug in dem durch den Stichkanal der Gewebeschicht laufenden Nähfaden entfaltet und flächig an den Rand des Stichkanals anlegt.

Aus der US 5,053,046 A ist ein Verschlußelement insbesondere im Zusammenhang mit der Versiegelung von Stichkanälen im Rückenmark bekannt, welches einen Haltekegel aufweist, an dem Flügel sitzen. Die Flügel sind aus einem elastischen Material hergestellt und können sich aufgrund ihrer intrinsischen Öffnungselastizität ausfalten.

Verschlußvorrichtungen für Gewebeöffnungen sind auch aus der WO 95/29635 A1 oder der US 5,944,730 A bekannt.

Davon ausgehend liegt der Erfindung die Aufgabe zugrunde, eine Verschlußvorrichtung der eingangs genannten Art zu schaffen, welche für einen Operateur auf einfache Weise einsetzbar ist.

Diese Aufgabe wird bei der eingangs genannten Verschlußvorrichtung erfindungsgemäß dadurch gelöst, daß die Flügel über jeweilige Gelenke schwenkbar an einem Basisteil gehalten sind.

Dadurch, daß erfindungsgemäß die Flügel über jeweilige Gelenke schwenkbar gehalten sind, läßt sich auf einfache Weise eine Anlagestellung erreichen, in der die Flügel oder von den Flügeln gehaltene Anlageelemente an das Gewebe anlegbar sind. Es läßt sich aber auch auf einfache Weise eine eingeschwenkte Stellung erreichen, in welcher die Querabmessungen der Verschlußvorrichtungen minimiert sind, so daß diese durch den Stichkanal und insbesondere durch eine Trokarhülse in eine entsprechende Körperhöhle einführbar ist, um die Flügel an das Gewebe anlegen zu können.

Der Übergang zwischen der eingeschwenkten Stellung und der ausgeklappten Anlagestellung läßt sich auf einfache Weise erreichen.

Das Basisteil läßt sich dann so ausbilden, daß es auf definierte Weise beispielsweise durch eine Trokarhülse durchführbar ist, um so insbesondere ein Verkanten zu vermeiden. Ein Operateur kann dann auf einfache Weise eine Positionierung der Verschlußvorrichtung herbeiführen; dies ist auch ohne optische Kontrolle möglich.

Eine entsprechende Verschlußvorrichtung läßt sich auf einfache Weise herstellen, indem beispielsweise die Gelenke über Filmscharniere ausgebildet sind.

Ganz besonders vorteilhaft ist es, wenn die Gelenke Scharniergelenke sind. Solche Scharniergelenke weisen eine einzige Schwenkachse auf. Bei der erfindungsgemäßen Verschlußvorrichtung genügt eine einzige Schwenkachse, da die Flügel aus einer eingeschwenkten Stellung, in welcher die äußeren Querabmessungen der Verschlußvorrichtungen minimiert sind, nur in eine Anlagestellung überführt werden müssen, in welcher die Verschlußvorrichtung mit den Flügeln an das Gewebe anlegbar ist.

Solche Scharniergelenke lassen sich auf einfache Weise als Filmscharniere herstellen. Ein Filmscharnier ist durch eine Materialverdünnung in dem Bereich, in dem die Schwenkbarkeit gewährleistet sein muß, charakterisiert.

Insbesondere sind dabei Schwenkachsen der Gelenke im wesentlichen rechtwinklig zu einer zentralen Achse des Basisteils orientiert. Dadurch läßt sich auf einfache Weise eine Minimierung der äußeren Querabmessung der Verschlußvorrichtung erreichen, in dem auf die zentrale Achse zu verschwenkt wird. Weiterhin läßt sich eine große Anlagefläche über die Flügel bereitstellen, indem von der zentralen Achse weg nach außen verschwenkt wird.

Günstig ist es ferner, wenn die Schwenkachsen der Gelenke parallel zu Tangenten an einen Außenumfang des Basisteils liegen. Dadurch läßt sich eine Maximierung der Anlagefläche der Verschlußvorrichtung an das Gewebe erreichen, wenn die Flügel ausgeschwenkt sind, das heißt in einer Anlagestellung liegen. Beispielsweise lassen sich die Flügel kreissektorförmig ausbilden, so daß der Zwischenbereich zwischen beabstandeten Flügeln minimiert ist, um so wiederum die Anlagefläche bei ausgeschwenkten Flügeln maximieren zu können.

Die erfindungsgemäße Verschlußvorrichtung läßt sich auf einfache Weise herstellen, wenn die Flügel einstückig an dem Basisteil gehalten sind. Es müssen dann insbesondere keine Wellen oder dergleichen zur Ausbildung der Gelenke vorgesehen werden.

Es kann vorgesehen sein, daß Flügel außerhalb der zugeordneten Gelenke im wesentlichen starr ausgebildet sind. Dadurch läßt sich eine definierte stabile, im wesentlichen starre Anlagefläche für die Anlage der Verschlußvorrichtung an dem Gewebe bereitstellen.

Es kann auch vorgesehen sein, daß die Flügel Anlageelemente aus einem biegbaren Material halten. Insbesondere ist dabei zwischen benachbarten Flügeln ein Anlageelement gespannt. Das Anlageelement kann dabei beispielsweise in der Art eines Filmscharniers an einem Flügel gehalten sein, wobei ein Flügel insbesondere leistenförmig ausgebildet ist. Es läßt sich dadurch eine Anlagefläche bereitstellen, welche unterbrechungsfrei ist. Bei entsprechender Anordnung der Anlageelemente an den Flügeln läßt sich in der ausgeklappten Stellung auch eine durchgehende glatte Anlagefläche bereitstellen, so daß beispielsweise das Einwachsen von Gewebe in Zwischenräume zwischen Flügeln verhindert ist. Die Anlageelemente können beispielsweise in der Art von Filmscharnieren an den Flügeln sitzen.

Aufgrund der biegbaren Ausbildung der Anlageelemente sind diese in der eingeklappten Stellung gefaltet, wobei Bereiche der Anlageelemente übereinanderliegen. In der eingeklappten Stellung läßt es sich dann erreichen, daß die Anlageelemente nicht über den Außenumfang des Basisteils hinausragen, während die Anlageflächen beim Übergang in die ausgeklappte Stellung sich ausbreiten lassen. Der Übergang von der eingeklappten Stellung in die ausgeklappte Stellung ist ähnlich zu dem Aufklappen eines Schirms.

Es ist vorteilhaft, wenn das Basisteil außerhalb der Gelenke im wesentlichen starr ausgebildet ist. Eine entsprechende Verschlußvorrichtung läßt sich auch auf einfache Weise herstellen.

Die erfindungsgemäße Verschlußvorrichtung läßt sich auf einfache Weise beispielsweise über eine Trokarhülse in eine Körperhöhle einbringen, wenn die Flügel so schwenkbar am Basisteil angeordnet sind, daß sie in einer eingeklappten Stellung nicht seitlich über das Basisteil hinausragen. Die Außenabmessungen der Verschlußvorrichtung sind dann in der eingeklappten Stellung eben durch die Außenabmessungen des Basisteils bestimmt. Die Verschlußvorrichtung läßt sich dann insbesondere verkantungsfrei durch die Trokarhülse in die Körperhöhle einfügen.

Weiterhin ist es günstig, wenn die Flügel in einer ausgeklappten Stellung Anlageflächen an das Gewebe bilden und/oder halten.

Für eine definierte Lage der Flüge bei der Anlage an das Gewebe ist es günstig, wenn diese in der ausgeklappten Stellung im wesentlichen rechtwinklig zu einer zentralen Achse des Basisteils orientiert sind. Es läßt sich dann eine stabile Anlagestellung erreichen, wobei beispielsweise verhinderbar ist, daß die Verschlußvorrichtung nach Entfernen der Trokarhülse in den Stichkanal hineingezogen wird. Auch bei sehr starkem Zug am Nahtmaterial zum Vernähen der Verschlußvorrichtung mit dem Gewebe ist sichergestellt, daß die Flügel in einer horizontalen Ebene verbleiben und nicht in die Trokarinzision hineingezogen werden.

Um eine leichte Einführbarkeit der Verschlußvorrichtung beispielsweise durch eine Trokarhülse in eine Körperhöhle zu erreichen, sind insbesondere die Gelenke an dem Basisteil gegenüber einem Umfangsrand des Basisteils zurückgesetzt. Dadurch läßt sich eine eingeklappte Stellung herstellen, bei welcher die Flügel nicht über den Umfangsrand hinausragen. Die Gelenke lassen sich dann auch so anordnen, daß die Flügel in der eingeklappten Stellung beispielsweise fächerartig angeordnet sind, um so eine Vielzahl von schwenkbaren Flügeln, welche eine große Anlagefläche bereitstellen, vorsehen zu können.

Weiterhin ist es günstig, wenn die Gelenke an einer Oberseite des Basisteils sitzen, welche bei am Gewebe anliegenden Anlageflächen dem Gewebe zugewandt ist.

Dadurch läßt sich sicherstellen, daß die Flügel in einer Anlagestellung (der ausgeklappten Stellung) eine im wesentlichen horizontale Ebene bilden, über die das Basisteil nicht hinausragt. Es wird dadurch verhindert, daß das Basisteil oder ein Teil des Basisteils in den Stichkanal gelangt und ein Zuwachsen des Stichkanals verhindert wird.

Zur Erzielung einer großen Anlagefläche ist es vorteilhaft, wenn mindestens zwei Flügel vorgesehen sind. Insbesondere sind diametral gegenüberliegende Flügel vorgesehen, um eine Verkantung zu vermeiden.

Es ist dann besonders vorteilhaft, wenn die Flügel um den Umfang des Basisteils angeordnet sind, um eine Verkantung in jeder Richtung zu vermeiden und eine große Anlagefläche bereitzustellen.

Es ist vorgesehen, daß das Basisteil einen kreisrunden Außenquerschnitt hat. Es läßt sich dann auf einfache Weise durch eine Trokarhülse in eine Körperhülse bringen, um den Stichkanal, in welchem die Trokarhülse sitzt, nachträglich zu verschließen.

Ganz besonders vorteilhaft ist es, wenn an dem Basisteil ein Nähfaden gehalten ist. Mit diesem Nähfaden läßt sich die Verschlußvorrichtung mit dem Gewebe vernähen, um so die Verschlußvorrichtung sicher zu halten und ein Schließen des Stichkanals (der Öffnung im Gewebe) zu erreichen.

Insbesondere weist das Basisteil beabstandete Durchgangsöffnungen für einen Nähfaden auf. Durch diese Durchgangsöffnung kann der Nähfaden geschleift werden, wobei zwischen den Durchgangsöffnungen gleichzeitig an dem Basisteil eine Anlagefläche für den Nähfaden bereitgestellt ist. Über diese Anlagefläche mit durchgeschleiftem Nähfaden kann dann das Basisteil gegen das Gewebe gezogen werden, um so eine sichere Fixierung zu gewährleisten.

Es kann vorgesehen sein, daß die Flügel in einer eingeklappten Stellung schräg zum Basisteil stehen. Dadurch ist ein hohlkegelartiger Raum gebildet, in den ein Öffnungsinstrument eingreifen kann, um die Flügel auseinanderzufalten und sie in die ausgeklappte Stellung zu bringen. Wenn die Flügel schräg zum Basisteil stehen, dann ist das Einfädeln des entsprechenden Instruments erleichtert. Es kann dabei vorgesehen sein, daß die Flügel in der eingeklappten Stellung aufgrund der Ausbildung der Gelenke schräg stehen. Es kann aber. auch sein, daß die eingeklappte Stellung eine Stellung ist, bei der die Flügel sich von einer im wesentlichen parallelen Stellung (bezogen auf die zentrale Achse des Basisteils) von selber ausfalten, bis sie an eine umgebende Wand, beispielsweise die Wand einer Trokarhülse, anstoßen und sich nicht weiter auseinanderfalten können. Auch in dieser eingeklappten Stellung (bezogen auf die ausgeklappte Stellung), in welcher die Flügel Anlageflächen für das Gewebe bereitstellen), läßt sich die Verschlußvorrichtung durch einen Stichkanal in einen Körperhohlraum einführen.

Ganz besonders vorteilhaft ist es, wenn das Basisteil eine oder mehrere Anlageflächen für die Flügel bereitstellt, welche das Schwenken der Flügel über eine Anlagestellung hinaus sperrt. Dadurch ist eine definierte Anlagestellung hergestellt, wobei diese Anlagestellung über die Anlageflächen an dem Basisteil auch stabilisiert ist. Über die Anlagefläche oder Anlageflächen an dem Basisteil läßt sich insbesondere verhindern, daß die Flügel des Implantats überschwenkt werden. Dadurch wiederum läßt sich gewährleisten, daß auch bei starkem Zug am Nahtmaterial die Flügel des Implantats in der entsprechenden Anlageebene verbleiben und die Verschlußvorrichtung insbesondere mit ihrem Basisteil nicht in den Stichkanal hineingezogen wird.

Beispielsweise sind die Anlagefläche oder Anlageflächen an einem ringförmigen Anlageelement gebildet, welches insbesondere ein Teil des Basisteils ist. Dadurch läßt sich die Anlagefläche auf einfache Weise herstellen.

Es kann auch vorgesehen sein, daß die Flügel eine Abstützung zum Anlegen an die zugeordneten Anlageflächen aufweisen. Diese Abstützung erstreckt sich beispielsweise in einer Längsrichtung der Flügel. Mit einer solchen Abstützung läßt sich ein größerer Abstand zwischen dem Gelenk und der Anlagefläche herstellen, so daß beispielsweise eine verbesserte Schwenkbarkeit erreichbar ist oder die Gelenke auf einfachere Weise herstellbar sind.

Günstig ist es, wenn die Flügel eine sich vom dem Basisteil weg erweiternde Breite aufweisen, und beispielsweise eine ringsektorförmige Außenrandgestalt aufweisen. Die Flügel lassen sich dann um ein kreisförmiges Basisteil anordnen, ohne daß sie über dieses seitlich hinausragen (in der eingeklappten Stellung). Sie lassen sich dann in der eingeklappten Stellung so positionieren, daß sie um eine zentrale Achse des Basisteils herum angeordnet sind. Beim Ausfalten wird eine große Anlagefläche bereitgestellt, die Lücken aufgrund des Abstands zwischen benachbarten Flügel aufweist, wobei diese Lücken wiederum eine minimale Fläche aufweisen.

Günstig ist es, wenn das Basisteil mit einer Ankopplung für einen Haltedorn versehen ist. Ein solcher Haltedorn läßt sich als Navigationsinstrument einsetzen, um die erfindungsgemäße Verschlußvorrichtung an dem Gewebe zu positionieren. Aufgrund der erfindungsgemäßen Ausgestaltung der Verschlußvorrichtung mit Basisteil und Flügeln läßt sich über den Haltedorn die Verschlußvorrichtung (in der eingeklappten Stellung) in die Körperhöhle führen, ohne daß eine optische Kontrolle notwendig ist.

Es kann dabei vorgesehen sein, daß das Basisteil ein Halteelement für die Flügel und ein Ringelement aufweist. An dem Halteelement sitzen die Flügel. Das Ringelement stellt die Anlageflächen dar, um ein Verschwenken der Flügel über die Anlagestellung hinaus zu verhindern.

Eine entsprechende Verschlußvorrichtung läßt sich auf einfache Weise herstellen, wenn das Ringelement an dem Halteelement in der Art eines Schnappverschlusses gehalten ist; das Ringelement läßt sich dann an dem Halteelement auf einfache Weise fixieren, wobei nach der Fixierung eine sichere Verbindung zwischen Ringelement und Halteelement gewährleistet ist.

Der Erfindung liegt ferner die Aufgabe zugrunde, eine Applikatorvorrichtung der eingangs genannten Art bereitzustellen, mit welcher sich auf einfache und sichere Weise eine erfindungsgemäße Verschlußvorrichtung positionieren läßt.

Diese Aufgabe wird bei der eingangs genannten Applikatorvorrichtung erfindungsgemäß dadurch gelöst, daß ein in der Trokarhülse längsverschiebliches Positionierelement vorgesehen ist, mittels welchem die Flügel der Verschlußvorrichtung von einer eingeklappten Stellung, in welcher die Verschlußvorrichtung in einer Trokarhülse verschieblich ist, in eine ausgeklappte Stellung bringbar sind.

Das Positionierelement dient dazu, die Flügel auszufalten, das heißt in die Anlagestellung zu bringen. Es muß dann keine Eigenelastizität der Gelenke vorgesehen werden, das heißt es muß kein Eigenausfalten der Flügel vorgesehen werden. Über das Vorsehen eines längsverschieblichen Positionierelements läßt sich die Verschlußvorrichtung ohne optische Einstellhilfen positionieren.

Insbesondere weist das Positionierelement Anlageflächen für die Flügel auf, um diese auszuschwenken.

Weiterhin ist es vorteilhaft, wenn ein Haltedorn zum Halten oder Positionieren der Verschlußvorrichtung vorgesehen ist. Dieser Haltedorn dient zum Navigieren der Verschlußvorrichtung bei dem Einbringen in die Körperhöhle und bei der Fixierung an dem Gewebe.

Es ist dann besonders vorteilhaft, wenn das Positionierelement den Haltedorn zumindest teilweise umgibt. Dadurch lassen sich Positionierelement und Haltedorn getrennt betätigen. Beispielsweise ist das Positionierelement bei dem Einschieben der Verschlußvorrichtung in eine Körperhöhle nicht notwendig, sondern erst wenn die Flügel geöffnet werden sollen. Wenn das Positionierelement den Haltedorn umgibt, dann läßt sich der Haltedorn in der Trokarhülse ungehindert bewegen.

Es ist dann besonders vorteilhaft, wenn der Haltedorn längsverschieblich an dem Positionierelement geführt ist. Das Positionierelement stellt dann selber die Führung, insbesondere über einen Führungskanal, für den Haltedorn bereit. Dadurch läßt sich eine sichere Positionierung des Verschlußelementes insbesondere unter Minimierung von Querbewegungen erreichen.

Es ist dann besonders vorteilhaft, wenn das Positionierelement eine Zentrierung für den Haltedorn bereitstellt, um so ohne optische Einstellhilfen die Verschlußvorrichtung in die Körperhöhle bringen zu können und dort auffalten zu können.

Weiterhin ist es günstig, wenn ein Nähfaden durch den Haltedorn geführt ist. Dadurch läßt sich unter Minimierung von Zugkräften quer zu der zentralen Achse des Basisteils die Verschlußvorrichtung über den Nähfaden an das Gewebe drücken.

Besonders vorteilhaft ist es, wenn eine Reduzierhülse oder ein Satz von Reduzierhülsen vorgesehen ist, um das Positionierelement zu positionieren. Diese Reduzierhülsen füllen den Zwischenraum zwischen einem Innenraum der Trokarhülse und dem Positionierelement aus. Durch die Reduzierhülsen läßt sich auch bei unterschiedlichem Durchmesser der Trokarhülse eine vorgegebene Verschlußvorrichtung mit zugehörigem Positionierelement positionieren. Gleichzeitig läßt sich eine Zentrierung des Positionierelementes und damit wiederum der Verschlußvorrichtung beim Einschieben durch die Trokarhülse erreichen. Dadurch kann auf optische Einstellhilfen bei der Fixierung der Verschlußvorrichtung an dem Gewebe verzichtet werden.

Die nachfolgende Beschreibung bevorzugter Ausführungsformen dient im Zusammenhang mit der Zeichnung der näheren Erläuterung der Erfindung. Es zeigen:
- Figur 1: eine schematische Schnittansicht eines Ausführungsbeispiels einer erfindungsgemäßen Applikatorvorrichtung beim Durchschieben eines Ausführungsbeispiels einer erfindungsgemäßen Verschlußvorrichtung;
- Figur 2: die Applikatorvorrichtung gemäß Figur 1, wobei die Verschlußvorrichtung in einer Körperhöhle positioniert ist und Flügel der Verschlußvorrichtung aufgefaltet werden;
- Figur 3: die Applikatorvorrichtung gemäß Figur 1 bei ausgeklappten Flügeln der Verschlußvorrichtung, welche an die Körperhöhle begrenzendes Gewebe anliegen;
- Figur 4: eine schematische Darstellung eines Ausführungsbeispiels einer erfindungsgemäßen Verschlußvorrichtung in einer eingeklappten Stellung der Flügel;
- Figur 5: die Verschlußvorrichtung gemäß Figur 4 mit ausgeklappten Flügeln;
- Figur 6: eine Teilschnittansicht eines Ausführungsbeispiels eines Basisteils, welches Flügel hält,
- Figur 7: eine Ausführungsform von Flügeln;
- Figur 8: ein perspektivische Ansicht einer weiteren Ausführungsform eines Flügels;
- Figur 9: ein weiteres Ausführungsbeispiel einer erfindungsgemäßen Verschlußvorrichtung in der eingeklappten Stellung; und
- Figur 10: die Verschlußvorrichtung gemäß Figur 10 in der ausgeklappten Stellung.

Erfindungsgemäße Verschlußvorrichtungen werden zum Verschließen von Öffnungen und insbesondere von Stichkanälen 10 in einer Gewebeschicht 12 eingesetzt. Die Gewebeschicht 12 begrenzt dabei eine Körperhöhle 14 wie beispielsweise den Bauchraum.

In dem Stichkanal 10 der Gewebeschicht 12 sitzt eine Trokarhülse 16 mit einem spitzen Ende 18. Die Gewebeschicht 12, welche den Stichkanal 10 umgibt, umfaßt im Falle des Bauchraums als Körperhöhle 14 eine Oberhautschicht 20, eine Fettschicht 22, eine Muskelschicht 24, die Faszie 26 und das Peritoneum 28.

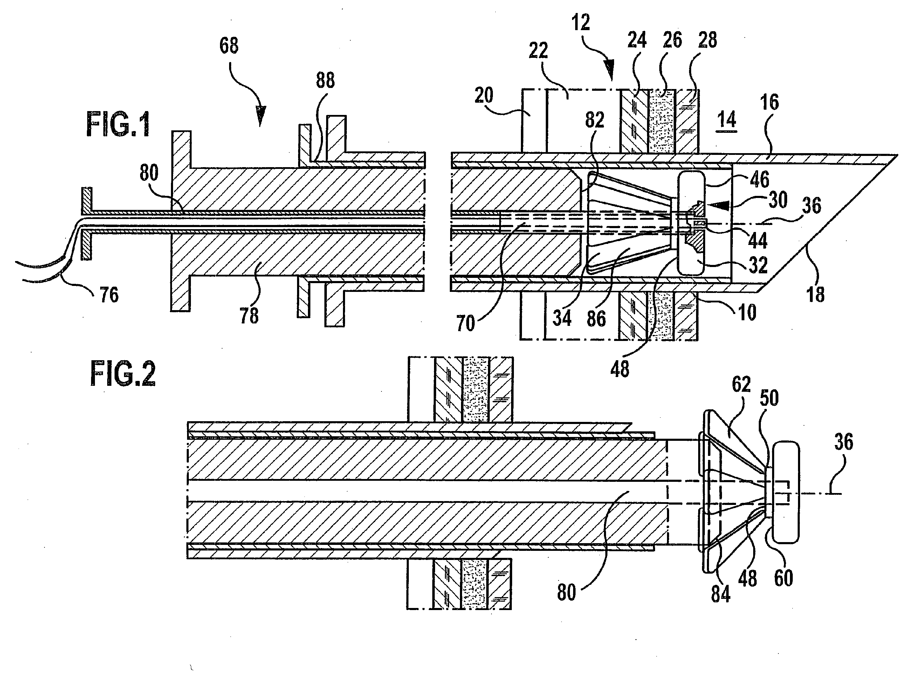

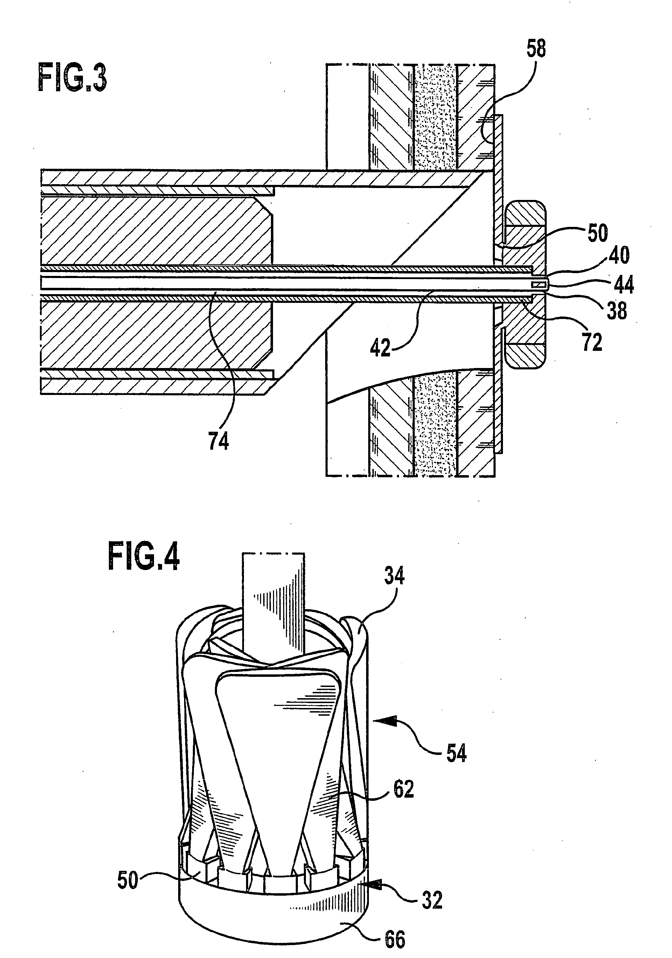

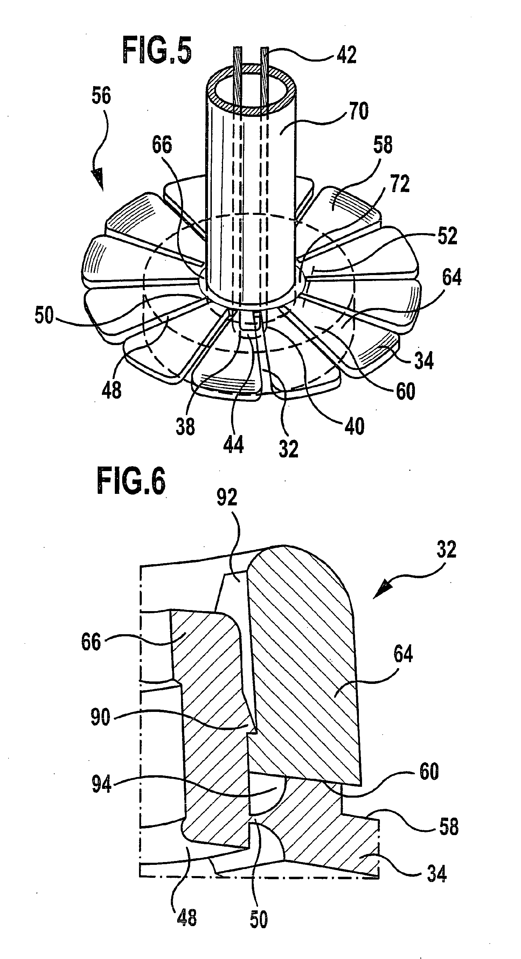

Ein Ausführungsbeispiel einer erfindungsgemäßen Verschlußvorrichtung, welche in Figur 1 als Ganzes mit 30 bezeichnet ist, umfaßt ein Basisteil 32, an welchem eine Mehrzahl von Flügeln 34 sitzen (Figuren 4, 5). Das Basisteil 32 weist einen kreisförmigen Außenquerschnitt auf mit einer zentralen (Mittelpunkt-)Achse 36. Parallel zu dieser zentralen Achse 36 versetzt zu dieser sind zwei beabstandete Durchgangsöffnungen 38, 40 in dem Basisteil 32 gebildet (Figur 3). Durch diese Durchgangsöffnungen 38, 40 ist ein Nähfaden 42 durchgefädelt, wobei bei gespanntem Nähfaden 42 ein Nähfadenbereich 44 an einer Unterseite 46 des Basisteils 32 anliegt. Die Unterseite des Basisteils 46 hat eine im wesentlichen ebene Oberfläche und kann mit einer Vertiefung für den Nähfadenbereich 44 versehen sein, so daß der Nähfaden nicht über diese ebene Oberfläche hinausragt beziehungsweise zumindest versenkt anliegt.

An einer der Unterseite 46 gegenüberliegenden Oberseite 48 des Basisteils 32 sitzen schwenkbar gehalten die Flügel 34. Jedem Flügel 34 ist dabei als Schwenkgelenk ein Scharniergelenk 50 zugeordnet, bei dem es sich beispielsweise um ein Filmscharnier handelt, um die Schwenkbarkeit der Flügel 34 zu gewährleisten. Eine Schwenkachse 52 (Figur 5) ist dabei quer und insbesondere rechtwinklig zu der zentralen Achse 36 des Basisteils orientiert. Die Schwenkachsen 52 verlaufen parallel zu der entsprechenden Tangente an den Außenumfang des Basisteils 32. Die Scharniergelenke 50 sind dabei soweit bezüglich eines Außenumfangs des Basisteils 32 zurückgesetzt, daß in einer eingeklappten Stellung 54 (Figur 4) der Flügel 34, bei welcher die Flügel 34 von der Oberseite 48 weg verschwenkt sind, nicht über den Außenumfang des Basisteils 32 hinausragen, so daß die seitlichen Abmessungen der Verschlußvorrichtung 30 durch das Basisteil 32 bestimmt sind.

In einer ausgeklappten Stellung 56 (Figuren 3, 5) sind die Flügel 34 auf die Oberseite 48 zu verschwenkt und liegen dabei insbesondere rechtwinklig zu der zentralen Achse 36.

Dadurch sind durch die Flügel 34 Anlageflächen 58 bereitgestellt, über welche die Verschlußvorrichtung 30 an das den Stichkanal umgebendes Gewebe (in dem gezeigten Beispiel an das Peritoneum 28) anlegbar ist, um so diese Öffnung in der Gewebeschicht 12 zum Außenraum hin zu verschließen.

Die Flügel 34 sind um die zentrale Achse 36 herum angeordnet. Es sind dabei mindestens zwei Flügel 34 und vorzugsweise eine derartige Anzahl von Flügel 34 vorgesehen, daß eine möglichst große Anlagefläche an die Gewebeschicht 12 bereitgestellt ist. Insbesondere sind diametral gegenüberliegenden Flügel 34 vorgesehen.

Die Flügel 34 selber weisen, wie in Figur 5 gezeigt, eine solche Gestalt auf, daß sie von dem Basisteil weg einen sich erweiternden Breitequerschnitt aufweisen. Die Flügel 34 sind dadurch zumindest näherungsweise kreissektorförmig ausgebildet. Dadurch läßt sich eine große Anlagefläche zum Anlegen an die Gewebeschicht 12 bereitstellen. Es ist dadurch auch möglich, in der eingeklappten Stellung 54 (Figur 4) zu gewährleisten, daß die Flügel 34 nicht über den Außenumfang des Basisteils 32 hinaus stehen.

Das Basisteil 32 stellt eine insbesondere ringförmige Anlagefläche 60 für die Flügel 34 bereit, welche beim Anliegen einer der Oberseite 48 zugewandten Seite 62 (welche der Anlageseite 58 gegenüberliegt) der Flügel 34 deren Weiterschwenkung sperrt. Die Anlagefläche 60 ist vorzugsweise an einem Ringelement 64 gebildet (Figur 6), welches ein Halteelement 66 des Basisteils 32 umgibt, wobei das Halteelement 66 wiederum die Flügel 34 über die Scharniergelenke 50 schwenkbar hält.

Die Anlagefläche 60 des Ringelements 64 ist vorzugsweise so ausgebildet, daß in der ausgeklappten Stellung 56 die Flügel im wesentlichen rechtwinklig zu der zentralen Achse 36 des Basisteils 32 orientiert sind; auf diese Weise können die Flügel 34 flach an der Gewebeschicht 12 anliegen.

Es kann vorgesehen sein, daß, wie in Figur 1 gezeigt, die Flügel 34 in der eingeklappten Stellung 54 in einem Winkel zu der zentralen Achse 36 orientiert sind, das heißt schräg zu dieser orientiert sind. Dadurch läßt sich, wie unten noch näher beschrieben wird, auf einfache Weise die Überführung aus der eingeklappten Stellung 54 in die ausgeklappte Stellung 56 der Flügel 34 mittels einer Applikatorvorrichtung erreichen.

Die erfindungsgemäße Verschlußvorrichtung 30 läßt sich über eine erfindungsgemäße Applikatorvorrichtung, von der ein Ausführungsbeispiel in den Figuren 1 bis 3 als Ganzes mit 68 bezeichnet ist, positionieren. Die Applikatorvorrichtung 68 umfaßt dazu einen Haltedorn 70. Das Basisteil 32 weist an dem Halteelement 66 eine Ankopplung 72 für diesen Haltedorn 70 auf (Figuren 3, 5). Das Basisteil 32 läßt sich über diese Ankopplung 72 an dem Haltedorn 70 fixieren. Dadurch wiederum kann die Verschlußvorrichtung über den Haltedorn 70 zur Anlage an der Gewebeschicht 12 navigiert werden.

Der Haltedorn 70 weist einen hohlen Innenraum 74 auf, durch welchen der Nähfaden 42 nach außen geführt ist. Es läßt sich dann mittels Nadeln 76 nach Position der Verschlußvorrichtung 30 an der Gewebeschicht 12 und Entfernung der Trokarhülse 16 aus dem Stichkanal 10 die Verschlußvorrichtung 30 mit der Gewebeschicht 12 vernähen.

Der Haltedorn 70 ist in einem Positionierelement 78 längsverschieblich geführt. Das Positionierelement 78 weist dazu einen Längskanal 80 auf, dessen Innendurchmesser an einen Außendurchmesser des Haltedorns 70 angepaßt ist. Dadurch läßt sich auch eine Zentrierung des Haltedorns 70 in dem Positionierelement 78 erreichen, um dessen Querbeweglichkeit relativ zu dem Positionierelement 78 zu sperren.

Das Positionierelement 78 wiederum ist verschieblich in der Trokarhülse 16 geführt, um so bei entsprechender Verschiebung auf die Flügel 34 in deren eingeklappten Stellung 54 wirken zu können, um die ausgeklappte Stellung 56 herzustellen.

Das Positionierelement 78 weist dazu an seinem distalen Ende 82 einen kegelstumpförmigen Rand 84 auf. Das Positionierelement 78 selber weist vorzugsweise eine zylindrische Außengestalt auf. Der kegelstumpfförmige Rand 84 ist dabei an eine Öffnungsweite der Flügel 34 in der eingeklappten Stellung 54 angepaßt. Dadurch kann das distale Ende 82 in einen Fächerraum 86 der Verschlußvorrichtung 30 eingeschoben werden, ohne an die Flügel 34 zu stoßen. Wird jedoch das Positionierelement 78 weiter in Richtung des Basisteils 32 verschoben, welches durch den Haltedorn 70 gehalten ist, dann lassen sich dadurch die Flügel 34 in Richtung der Unterseite 46 verschwenken, um die ausgeklappte Stellung 56 herzustellen, sofern die Verschwenkbarkeit der Flügel 34 nicht gesperrt ist.

Um die Applikatorvorrichtung 68 mit der Verschlußvorrichtung 30 an Trokarhülsen 16 eines vorgegebenen Durchmessers anpassen zu können, kann ein Satz von Reduzierhülsen vorgesehen sein, wobei eine Reduzierhülse in die Trokarhülse 16 eingeschoben ist. Das Positionierelement 78 ist dann wiederum in die Reduzierhülse 78 eingeschoben, wobei der Außendurchmesser des Positionierelements 78 an den Innendurchmesser der Reduzierhülse 88 angepaßt ist. Dadurch wiederum läßt sich eine Zentrierung des Positionierelements 78 in die Trokarhülse 16 erreichen, das heißt eine Querbewegung des Positionierelements 78 in der Trokarhülse 16 läßt sich sperren. Die Reduzierhülse 88 füllt damit im wesentlichen den Zwischenraum zwischen dem Positionierelement 78 und der Trokarhülse 16 aus, wenn das Positionierelement 78 einen kleineren Außendurchmesser aufweist, als es dem Innendurchmesser der Trokarhülse 16 entspricht.

Die erfindungsgemäße Applikatorvorrichtung 68 und die erfindungsgemäße Verschlußvorrichtung 30 funktionieren wie folgt:

Die Verschlußvorrichtung 30 wird über ihr Basisteil 32 an dem Haltedorn 70 fixiert. An dem Basisteil 32 ist der Nähfaden 44 gehalten, welcher durch den Innenraum 74 des Haltedorns 70 läuft. Am Ende des Nähfadens 44 sitzen die Nadeln 76. Der Haltedorn 70 ist dabei in dem Positionierelement 78 angeordnet. Die Applikatorvorrichtung 68 mit der gehaltenen Verschlußvorrichtung 30 wird in die Trokarhülse 16 eingeschoben, wobei die Flügel 34 in der eingeklappten Stellung 54 sind, das heißt sie ragen nicht über den Außenumfang des Basisteils 32 hinaus, so daß die Verschlußvorrichtung 30 über die Applikatorvorrichtung 68 in der Trokarhülse 16 verschiebbar ist.

Die Verschlußvorrichtung 30 wird dann in die Körperhöhle 14 so weit verschoben, daß die Flügel 34 aus der eingeklappten Stellung 54 in die ausgeklappte Stellung 56 bringbar sind. Die Schwenkbewegung wird durch das Positionierelement 78 bewirkt, welches in Richtung des Basisteils verschoben wird. Das distale Ende 82 des Positionierelements 78 wirkt auf die Flügel 34 und bewirkt deren Verschwenkung zu der Unterseite 46 des Basisteils 32 hin. Durch Anlage der Flügel 34 an die Anlagefläche 60 des Ringelementes 64 ist die ausgeklappte Stellung 56 erreicht. Die Trokarhülse 16 und das Positionierelement 78 werden dann zurückgeschoben, bis die Flügel 34 mit ihren Anlageflächen 58 an der Gewebeschicht 12 anliegen. Die Applikatorvorrichtung 68 und die Trokarhülse 16 lassen sich danach aus dem Stichkanal 10 entfernen.

Der Haltedorn 70 und das Positionierlement 78 sind vorzugsweise mit Längsöffnungen versehen, um den Nähfaden 42 auszufädeln. Im Stichkanal 10 verbleibt dann nach Entfernung der Trokarhülse 16 lediglich der Nähfaden 42, bei dem beispielsweise doppelt armiertes Nahtmaterial verwendet wird. Der Stichkanal 10 schließt sich und durch Zug am Nähfaden 42 läßt sich der Stichkanal innenseitig, das heißt an der Körperhöhle 14 durch die Verschlußvorrichtung 30 überdecken, wobei die Verschlußvorrichtung 30 wiederum durch Zug an die Gewebeschicht 12 angelegt wird. Beispielsweise werden in dieser Lage Enden des Nähfadens 42 verknotet, wobei der dann der gebildete Knoten in der Oberhautschicht 20 angeordneten Fettschicht 22 positioniert ist. Mit Hilfe der Nadeln 76 werden dann die Enden durch einen vom Stichkanal 10 nach außen geführten Einstich in die Fettschicht 22 und durch die Oberhautschicht 20 hindurch außenseitig verknotet, so daß der obere Teil der Fettschicht und die Oberhautschicht 20 gegeneinander gezogen werden. Der Stichkanal 10 wird dadurch auch außenseitig verschlossen.

Beim Übergang der Verschlußvorrichtung 30 in die Körperhöhle 14 können sich die Flügel 34 bereits ausfalten, wobei die endgültige ausgeklappte Stellung 56 durch das Positionierelement 78 erzeugt wird. Mit Hilfe des Haltedorns 70 kann die Verschlußvorrichtung 30 als Implantat genau auf den Stichkanal 10, das heißt die Trokarinzision navigiert werden. Da die Verschlußvorrichtung 30 über ihr Basisteil 32 an den Stichkanal 10 über den Innenraum der Trokarhülse 16 angepaßt ist, und da eine Zentrierung in der Trokarhülse 16 erreicht ist, läßt sich die Öffnung 10 in der Gewebeschicht 12 vollständig abdecken.

Über die Anlagefläche 60 des Ringelements 64 wird der Bereich der Scharniergelenke 50 sowie derjenige Bereich der Flügel 34, welcher mit den Scharniergelenken 50 verbunden ist beziehungsweise an dem diese Scharniergelenke 50 gebildet sind, stabilisiert. Dadurch ist gewährleistet, daß die Verschlußvorrichtung 30 auch bei starkem Zug an dem Nähfaden 42 in einer Stellung verbleibt, in der die Flügel 34 im wesentlichen rechtwinklig zu der zentralen Achse 36 des Basisteils 32 orientiert sind. Insbesondere läßt sich verhindern, daß die Verschlußvorrichtung 30 in den Stichkanal 10 hineingezogen wird.

Aufgrund der Zentrierung der Verschlußvorrichtung 30 in der Trokarhülse 16 über die erfindungsgemäße Applikatorvorrichtung 68 und aufgrund der schwenkbaren Anordnung der Flügel 34 bei der Verschlußvorrichtung 30 und insbesondere Vorsehen der Anlagefläche 60 ist keine optische Kontrolle der Positionierung der Verschlußvorrichtung 30 zur Anlage an die Gewebeschicht 12 erforderlich.

Vorzugsweise sind die Flügel 34 einstückig an dem Basisteil 34 gehalten.

Bei dem in Figur 6 gezeigten Ausführungsbeispiels eines Basisteils 32 sitzen die Flügel 34 einstückig über jeweilige Filmscharniere als Scharniergelenke 50 an dem Halteelement 66. Das Ringelement 64 ist ein getrenntes Element, welches das Halteelement 66 umgibt. Das Halteelement 66 ist mit um dessen Umfang verteilten Nasen 90 versehen, wobei das Ringelement 64 an dem Halteelement 66 in der Art eines Schnappverschlusses gehalten ist. Dazu weist das Ringelement 64 entsprechende Ausnehmungen 92 auf, in welche die Nasen 90 einsetzbar sind. Das Halteelement 66 und/oder das Ringelement 64 sind dabei so entsprechend elastisch ausgebildet, daß ein Eintauchen dieser Nasen 90 in die Ausnehmungen 92 ermöglicht ist, um diese beiden Elemente 64 und 66 miteinander zu fixieren, ein Austauchen über die Nasen 90 ist gesperrt.

Ansonsten ist das Basisteil 32 außerhalb des Scharniergelenkes 50 im wesentlichen starr ausgebildet. Ebenso sind die Flügel 34 außerhalb des Scharniergelenkes im wesentlichen starr ausgebildet, so daß eine Beweglichkeit der Flügel 34 relativ zu dem Basisteil 32 nur über die Scharniergelenke 50 erlaubt ist.

Die Scharniergelenke 50 sind durch eine Materialverdünnung gebildet: Ein dem Halteelement 66 zugewandtes Ende 94 der Flügel 34 weist eine bestimmte Breite auf. Im Bereich des Scharniergelenks 50 verschmälert sich der jeweilige Flügel 34 im Übergang zu dem Halteelement 66.

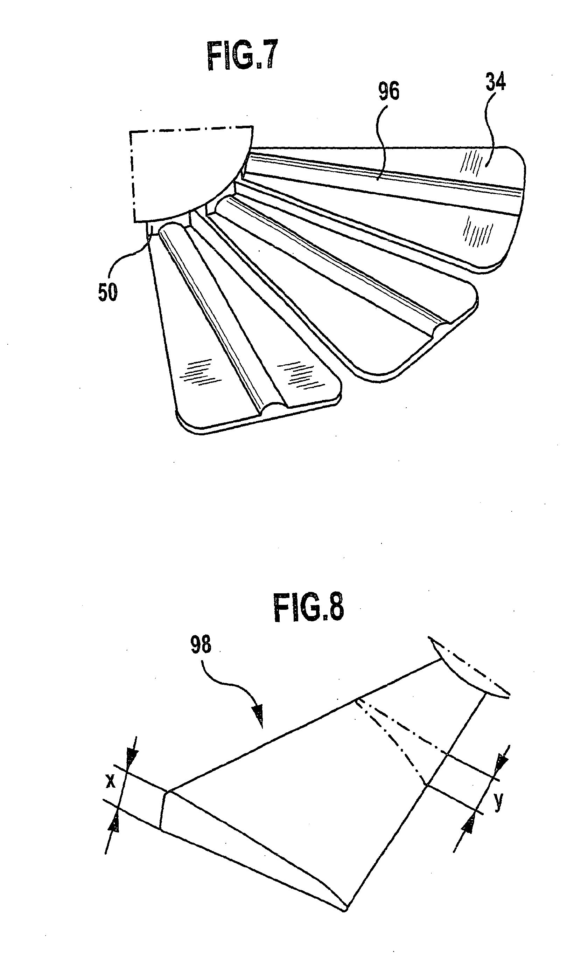

Es kann auch vorgesehen sein, wie in Figur 7 gezeigt, daß die Flügel 34 mit einer zusätzlichen Abstützung 96 versehen sind, welche insbesondere längs der jeweiligen Flügel 34 symmetrisch an deren die Anlagefläche 58 bildenden Oberseite verläuft. Diese Abstützung 96 erhöht die Stabilität der Flügel 34. Sie dient auch als Anlageelement der Flügel 34 an die Anlagefläche 68 des Ringelementes 64. Es läßt sich dadurch ein größerer Abstand zwischen den Enden 94 der Flügel 34 zu dem Ringelement 64 erreichen, das heißt die Scharniergelenke 50 lassen sich mit einem größeren Abstand zu diesen Ringelement 64 ausbilden. Dadurch wiederum wird die Schwenkbarkeit der Flügel 34 verbessert.

Es kann auch vorgesehen sein, daß eine entsprechende Abstützung nur im Bereich eines Endes 94 eines Flügels 34 angeordnet ist.

Es kann vorgesehen sein, wie in Figur 8 angedeutet, daß ein Flügel 98, welcher schwenkbar an der Verschlußvorrichtung 30 sitzt, eine solche Höhe X in seinem Profilquerschnitt an seiner breiteren Seite aufweist, wie sie einer Krümmungshöhe Y an einer Unterseite eines solches Flügels 98 entspricht.

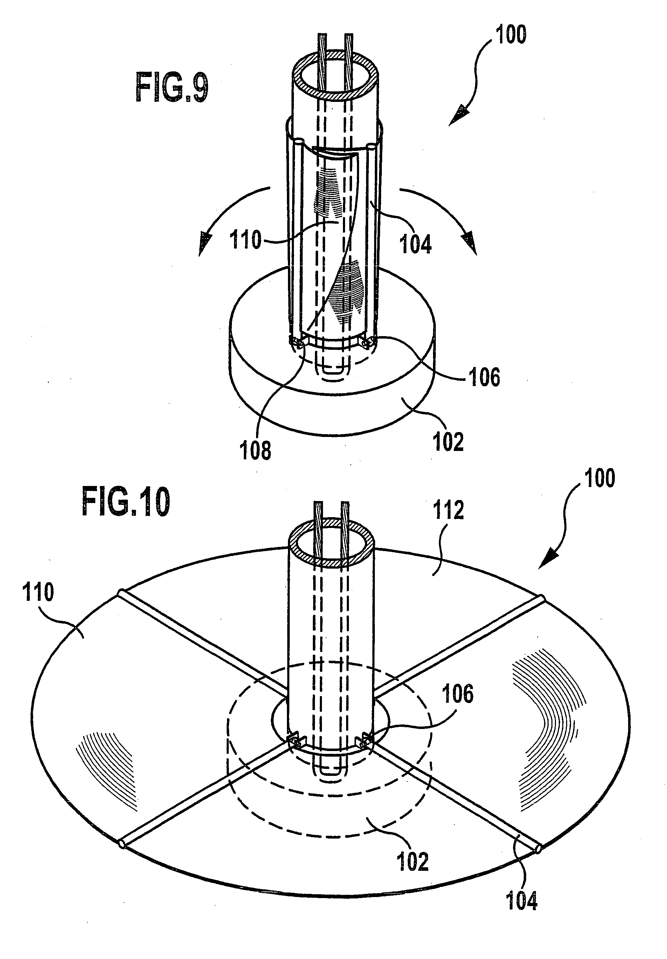

Bei einem weiteren Ausführungsbeispiel, welches in den Figuren 9 und 10 gezeigt und dort als Ganzes mit 100 bezeichnet ist, ist wiederum ein Basisteil 102 vorgesehen, welches grundsätzlich gleich ausgebildet ist wie das oben beschriebene Basisteil 32.

An diesem Basisteil 102 ist eine Mehrzahl von leistenförmig ausgebildeten Flügeln 104 gehalten. Diese Flügel sitzen an Scharniergelenken 106 schwenkbar an dem Basisteil 102. Bei den Scharniergelenken 106 kann es sich um Filmscharniere handeln. Es kann auch vorgesehen sein, daß der Flügel 104 bzw. ein Lagerelement 108 für den Flügel 104 mit Wellenstummeln versehen ist und das Lagerelement 108 bzw. der Flügel 104 Wellenaufnahmen für diese Wellenstummel aufweist.

Die Flügel 104 halten Anlageelemente 110 aus einem biegbaren Material und insbesondere Kunststoffmaterial. Die Anlageelemente 110 stellen Anlageflächen 112 bereit, um in einer ausgeklappten Stellung (Figur 10) die Verschlußvorrichtung 100 an Gewebe anlegen zu können. Die Anlageelemente 110 können zwischen benachbarten Flügel 104 aufgespannt sein. Sie können an einander zugewandten Seiten der benachbarten Flügel 104 angeordnet sein. Es ist aber auch möglich, daß die Anlageelemente 110 an einer Oberseite der Flügel 104 fixiert sind.

In einer eingeklappten Stellung (Figur 9) sind die Anlageelemente 110 so gebogen und insbesondere so gefaltet, daß sie nicht über den Außenumfang des Basisteils 102 hinausragen. Durch die Biegbarkeit des Materials der Anlageelemente 110 ist sichergestellt, daß von der eingeklappten Stellung in die ausgeklappte Stellung (Figur 10) übergegangen werden kann.

In der ausgeklappten Stellung liegen die Anlageflächen 112 im wesentlichen senkrecht zu der zentralen Achse des Basisteils 102 und bilden eine im wesentliche unterbrechungsfreie Anlagefläche auf. Wenn die Anlageelemente 110 an einer Oberseite der Flügel 104 gehalten sind, dann ist diese Anlagefläche auch glatt.

Die Anlageelemente 110 sind vorzugsweise so ausgebildet, daß in der ausgeklappten Stellung der Flügel 104 die Anlageflächen 112 zwischen benachbarten Flügeln 104 eben sind.

Die Anlageelemente 110 sind beispielsweise mittels einer Filmscharnierverbindung an den jeweiligen Flügeln 104 angeordnet.

Die erfindungsgemäße Verschlußvorrichtung 100 funktioniert grundsätzlich gleich wie oben anhand des ersten Ausführungsbeispiels beschrieben. Über die erfindungsgemäße Applikatorvorrichtung läßt sich die Verschlußvorrichtung 100 an einer zu verschließenden Gewebeöffnung positionieren. Das der Verschlußvorrichtung 100 zugeordnete Positionierelement ist entsprechend ausgebildet, um eine Ausfaltung der Flügel 104 bewirken zu können.

In Figur 9 ist eine Anlagestellung der Flügel 104 an einen Haltedorn gezeigt. Vorzugsweise sind die Anlageelemente 110 so ausgebildet, daß diese Stellung nur unter Kraftausübung erreichbar ist. Dies bedeutet, daß aufgrund der Eigenelastizität der Anlageelemente 110 diese in einer nicht kraftbeaufschlagten Stellung die Flügel 104 von dem Haltedorn weg bewegen, so daß die Flügel 104 in einem kleinen Winkel zu einer zentralen Achse des Basisteils 106 liegen. Dadurch kann dann das Positionierelement der Applikatorvorrichtung auf die Flügel 104 einwirken, um die ausgeklappte Stellung (Figur 10) herzustellen.

## Patentansprüche

1. Verschlußvorrichtung für eine Öffnung (10) in einer Gewebeschicht (12), mit einer Mehrzahl von Flügeln (34; 104), welche Anlageflächen (58; 112) an die Öffnung (10) umgebendes Gewebe bereitstellen und/oder halten, **dadurch gekennzeichnet, daß** die Flügel (34; 104) über jeweilige Gelenke (50; 106) schwenkbar an einem Basisteil (32; 102) gehalten sind.

2. Verschlußvorrichtung nach Anspruch 1, **dadurch gekennzeichnet, daß** die Gelenke (50; 106) Scharniergelenke sind.

3. Verschlußvorrichtung nach Anspruch 1 oder 2, **dadurch gekennzeichnet, daß** die Gelenke (50) Filmscharniere sind.

4. Verschlußvorrichtung nach einem der voranstehenden Ansprüche, **dadurch gekennzeichnet, daß** Schwenkachsen (52) der Gelenke (50) im wesentlichen rechtwinklig zu einer zentralen Achse (36) des Basisteils (32) orientiert sind.

5. Verschlußvorrichtung nach einem der voranstehenden Ansprüche, **dadurch gekennzeichnet, daß** die Schwenkachsen (52) der Gelenke (50) parallel zu Tangenten an einen Außenumfang des Basisteils (32) liegen.

6. Verschlußvorrichtung nach einem der vorangehenden Ansprüche, **dadurch gekennzeichnet, daß** die Flügel (34) einstückig an dem Basisteil (32) gehalten sind.

7. Verschlußvorrichtung nach einem der vorangehenden Ansprüche, **dadurch gekennzeichnet, daß** die Flügel (34) außerhalb der zugeordneten Gelenke (50) im wesentlichen starr ausgebildet sind.

8. Verschlußvorrichtung nach einem der Ansprüche 1 bis 7, **dadurch gekennzeichnet, daß** die Flügel (104) Anlageelemente (110) aus einem biegbaren Material halten.

9. Verschlußvorrichtung nach Anspruch 8, **dadurch gekennzeichnet, daß** zwischen benachbarten Flügeln (104) ein Anlageelement (110) gespannt ist.

10. Verschlußvorrichtung nach Anspruch 8 oder 9, **dadurch gekennzeichnet, daß** in einer eingeklappten Stellung die Anlageelemente (110) gefaltet sind.

11. Verschlußvorrichtung nach einem der vorangehenden Ansprüche, **dadurch gekennzeichnet, daß** das Basisteil (32; 102) außerhalb der Gelenke (50; 106) im wesentlichen starr ausgebildet ist.

12. Verschlußvorrichtung nach einem der vorangehenden Ansprüche, **dadurch gekennzeichnet, daß** die Flügel (34; 104) so schwenkbar am Basisteil (32; 102) angeordnet sind, daß sie in einer eingeklappten Stellung (54) nicht seitlich über das Basisteil hinausragen.

13. Verschlußvorrichtung nach einem der vorangehenden Ansprüche, **dadurch gekennzeichnet, daß** die Flügel (34; 104) in einer ausgeklappten Stellung (56) Anlageflächen (58) an das Gewebe bilden und/oder halten.

14. Verschlußvorrichtung nach Anspruch 13, **dadurch gekennzeichnet, daß** die Flügel (34; 104) in der ausgeklappten Stellung (56; 112) im wesentlichen rechtwinklig zu einer zentralen Achse (36) des Basisteils (32; 106) orientiert sind.

15. Verschlußvorrichtung nach einem der vorangehenden Ansprüche, **dadurch gekennzeichnet, daß** die Gelenke (50; 106) an dem Basisteil (32; 102) gegenüber einem Umfangsrand des Basisteils (32; 102) zurückgesetzt sind.

16. Verschlußvorrichtung nach einem der vorangehenden Ansprüche, **dadurch gekennzeichnet, daß** die Gelenke (50; 106) an einer Oberseite (48) des Basisteils (32; 102) sitzen, welche bei am Gewebe anliegenden Anlageflächen (58; 112) dem Gewebe zugewandt ist.

17. Verschlußvorrichtung nach einem der vorangehenden Ansprüche, **dadurch gekennzeichnet, daß** mindestens zwei Flügel (34; 104) vorgesehen sind.

18. Verschlußvorrichtung nach einem der vorangehenden Ansprüche, **dadurch gekennzeichnet, daß** diametral gegenüberliegende Flügel (34; 104) vorgesehen sind.

19. Verschlußvorrichtung nach einem der vorangehenden Ansprüche, **dadurch gekennzeichnet, daß** die Flügel (34; 104) um den Umfang des Basisteils (32; 102) angeordnet sind.

20. Verschlußvorrichtung nach einem der vorangehenden Ansprüche, **dadurch gekennzeichnet, daß** das Basisteil (32; 102) einen kreisrunden Außenquerschnitt aufweist.

21. Verschlußvorrichtung nach einem der vorangehenden Ansprüche, **dadurch gekennzeichnet, daß** an dem Basisteil (32; 102) ein Nähfaden (42) gehalten ist.

22. Verschlußvorrichtung nach einem der vorangehenden Ansprüche, **dadurch gekennzeichnet, daß** das Basisteil (32; 102) beabstandete Durchgangsöffnungen (38, 40) für einen Nähfaden (42) aufweist.

23. Verschlußvorrichtung nach einem der vorangehenden Ansprüche, **dadurch gekennzeichnet, daß** die Flügel (34) in einer eingeklappten Stellung (54) schräg zum Basisteil (32) stehen.

24. Verschlußvorrichtung nach einem der vorangehenden Ansprüche, **dadurch gekennzeichnet, daß** das Basisteil (32; 102) eine oder mehrere Anlageflächen (60) für die Flügel (34) bereitstellt, welche das Schwenken der Flügel (34) über eine Anlagestellung hinaus sperrt.

25. Verschlußvorrichtung nach Anspruch 24, **dadurch gekennzeichnet, daß** die Anlagefläche oder Anlageflächen (60) an einem ringförmigen Anlageelement (64) gebildet sind.

26. Verschlußvorrichtung nach Anspruch 24 oder 25, **dadurch gekennzeichnet, daß** die Flügel (34) eine Abstützung (96) zum Anlegen an die zugeordneten Anlageflächen (60) aufweisen.

27. Verschlußvorrichtung nach einem der vorangehenden Ansprüche, **dadurch gekennzeichnet, daß** die Flügel (34) eine sich von dem Basisteil (32) weg erweiternde Breite aufweisen.

28. Verschlußvorrichtung nach einem der vorangehenden Ansprüche, **dadurch gekennzeichnet, daß** das Basisteil (32; 102) mit einer Ankopplung (72) für einen Haltedorn (70) versehen ist.

29. Verschlußvorrichtung nach einem der vorangehenden Ansprüche, **dadurch gekennzeichnet, daß** das Basisteil (32) ein Halteelement (66) für die Flügel (34) und ein Ringelement (64) aufweist.

30. Verschlußvorrichtung nach Anspruch 29, **dadurch gekennzeichnet, daß** das Ringelement (64) an dem Halteelement (66) in der Art eines Schnappverschlusses gehalten ist.

31. Applikatorvorrichtung für eine Verschlußvorrichtung gemäß einem der vorangehenden Ansprüche, welche in eine Trokarhülse (16) einführbar ist, **gekennzeichnet durch** ein in der Trokarhülse (16) längsverschiebliches Positionierelement (78), mittels welchem die Flügel (34; 104) der Verschlußvorrichtung (30) von einer eingeklappten Stellung (54), in welcher die Verschlußvorrichtung in der Trokarhülse (16) verschieblich ist, in eine ausgeklappte Stellung (56) bringbar sind.

32. Applikatorvorrichtung für eine Verschlußvorrichtung nach Anspruch 31, **dadurch gekennzeichnet, daß** das Positionierelement (78) Anlageflächen für die Flügel (34; 104) aufweist, um diese auszuschwenken.

33. Applikatorvorrichtung für eine Verschlußvorrichtung nach Anspruch 31 oder 32, **dadurch gekennzeichnet, daß** ein Haltedorn (70) zum Halten und Positionieren der Verschlußvorrichtung (30) vorgesehen ist.

34. Applikatorvorrichtung für eine Verschlußvorrichtung nach Anspruch 33, **dadurch gekennzeichnet, daß** das Positionierelement (78) den Haltedorn (70) zumindest teilweise umgibt.

35. Applikatorvorrichtung für eine Verschlußvorrichtung nach Anspruch 33 oder 34, **dadurch gekennzeichnet, daß** der Haltedorn (70) längsverschieblich an dem Positionierelement (78) geführt ist.

36. Applikatorvorrichtung für eine Verschlußvorrichtung nach einem der Ansprüche 33 bis 35, **dadurch gekennzeichnet, daß** das Positionierelement (78) eine Zentrierung für den Haltedorn (70) bereitstellt.

37. Applikatorvorrichtung für eine Verschlußvorrichtung nach einem der Ansprüche 33 bis 36, **dadurch gekennzeichnet, daß** ein Nähfaden (42) durch den Haltedorn (70) geführt ist.

38. Applikatorvorrichtung für eine Verschlußvorrichtung nach einem der Ansprüche 31 bis 37, **dadurch gekennzeichnet, daß** eine Reduzierhülse (88) oder ein Satz von Reduzierhülsen vorgesehen ist, um das Positionierelement (78) zu positionieren.
